# EUROPEAN PATENT APPLICATION

(11) **EP 3 335 704 A1**
(43) Date of publication of application: **20.06.2018**
(21) Application number: 16204714.6
(22) Date of filing: 16.12.2016
(51) Int. Cl.: A61K 9/48, A61K 31/4162, A61P 3/10

(54) **PHARMACEUTICAL COMPOSITION COMPRISING OMARIGLIPTIN**

(71) Applicant: Hexal AG, 83607 Holzkirchen (DE)
(72) Inventor: BORN, Max, D-83607 Holzkirchen (DE); FISHER-DAUT, Diana, D-83607 Holzkirchen (DE); PUZIK, Andreas, D-83607 Holzkirchen (DE)
(74) Representative: Kluschanzoff, Harald

(57) **Abstract**

The present invention relates to a pharmaceutical composition comprising omarigliptin or a pharmaceutically acceptable salt thereof and at least one pharmaceutically acceptable excipient, wherein the pharmaceutical composition is in the form of a capsule for oral administration, and its use in the treatment of type 2 diabetes mellitus.

## Description

### FIELD OF THE INVENTION

The present invention relates to a pharmaceutical composition comprising omarigliptin or a pharmaceutically acceptable salt thereof and at least one pharmaceutically acceptable excipient, wherein the pharmaceutical composition is in the form of a capsule for oral administration, and its use in the treatment of type 2 diabetes mellitus.

### BACKGROUND OF THE INVENTION

Omarigliptin (2R,3S,5R)-2-(2,5-difluorophenyl)-5-[2-(methylsulfonyl)-2,6-dihydropyrrolo[3,4-c]pyrazol-5(4H)-yl]tetrahydro-2H-pyran-3-amine acts as a dipeptidyl peptidase IV (DPP IV) inhibitor and is used for the treatment of type 2 diabetes mellitus.

WO 2010/056708 describes omarigliptin and a pharmaceutical composition comprising omarigliptin, microcrystalline cellulose, croscarmellose sodium, and magnesium stearate. Omarigliptin, microcrystalline cellulose and croscarmellose are blended first. The mixture is then lubricated by magnesium stearate and pressed into tablets. Stability of the formulation has not been tested.

WO 2015/031228 describes a stable pharmaceutical composition comprising omarigliptin and neutral excipients in an amount of at least 75% by weight of the pharmaceutical formulation, wherein the neutral excipients comprise at least one non-reducible sugar diluent. The diluent is mannitol or microcrystalline cellulose or a mixture of mannitol and microcrystalline cellulose. Exemplified pharmaceutical formulations are tablets and consist essentially of omarigliptin, mannitol, microcrystalline cellulose, croscarmellose sodium, and magnesium stearate. The tablets are prepared by wet or dry processing methods. Diluents that should not be included in the pharmaceutical formulations described in WO 2015/031228 include lactose monohydrate, polyvinyl pyrrolidone, silicified microcrystalline cellulose, hydroxyethyl cellulose, anhydrous lactose, mannose, glucose, maltose, other reducing sugars, Neusilin (aluminum magnesium metasilicate), Kollidon (polyvinyl pyrrolidone), crospovidone and those with acid functional groups or basic functional groups. It is disclosed that one concern with developing a pharmaceutical composition of omarigliptin is the chemical stability of the compound. Omarigliptin is said to be sensitive to oxidation, hydrolysis (acid and base catalysed) and Maillard degradation (browning) with reducible sugar impurities in excipients.

While WO 2015/031228 claims to provide a stable pharmaceutical formulation of omarigliptin, the formulations disclosed therein entail significant limitations. Especially cumbersome is the limited choice with regard to the nature of the diluent. Furthermore, the required high amount of excipients results in low drug loading. Thus, there is a need for further stable omarigliptin formulations for oral administration.

### SUMMARY OF THE INVENTION

It has surprisingly been found that providing pharmaceutical compositions containing omarigliptin in capsule form increases stability of the pharmaceutically active ingredient omarigliptin compared to formulations described in the prior art.

Accordingly, the pharmaceutical composition of the present invention comprises omarigliptin or a pharmaceutically acceptable salt thereof and at least one pharmaceutically acceptable excipient, wherein the pharmaceutical composition is in the form of a capsule for oral administration.

Compared with the disclosure of WO 2015/031228, the present invention allows for provision of omarigliptin formulations with high flexibility regarding the choice of excipients and drug loading requirements, while at the same time minimizing degradation of omarigliptin, especially degradation via a Maillard reaction in the presence of commonly used excipients. Without being bound by theory, it can be postulated that this increased stability is mainly due to a decrease in the rate of the Maillard reaction when providing omarigliptin compositions in the form of a capsule for oral administration.

### DETAILED DESCRIPTION OF THE INVENTION

The pharmaceutical composition of the present invention comprises omarigliptin or a pharmaceutically acceptable salt thereof and at least one pharmaceutically acceptable excipient, wherein the pharmaceutical composition is in the form of a capsule for oral administration.

Capsules are a commonly used type of solid dosage form suitable for oral administration. Usually, the active pharmaceutically active ingredient, optionally in combination with at least one pharmaceutically acceptable excipient, is provided as a filling within a capsule shell. There are two main types of capsules, namely hard-shelled capsules and soft-shelled capsules. Both of these capsules are typically made from aqueous solutions of gelling agents, e.g. gelatin or polysaccharides. Capsules can be tailored to any specific need, e.g. by including plasticizers, coloring agents, preservatives, disintegrants and/or lubricants in the capsule material. In the present invention, any capsule useful for oral administration to a patient can be employed.

Preferably, the capsule for oral administration is a hard-shelled capsule or a soft-shelled capsule. While there is no limitation to the material of the capsule, except for its suitability for oral administration of pharmaceutically active agents such as omarigliptin or pharmaceutically acceptable salts thereof, gelatin-based capsules are preferred. Thus, in a preferred embodiment of the present invention, the capsule is a hard gelatin capsule. In another preferred embodiment of the present invention, the capsule is a soft gelatin capsule.

Regarding the at least one pharmaceutically acceptable excipient, no particular limitation exists provided suitable compositions are obtained. In various embodiments, the at least one pharmaceutically acceptable excipient comprises neutral excipients.

When the capsule according to the present invention is a hard-shelled capsule, preferably a hard gelatin capsule, omarigliptin or a pharmaceutically acceptable salt thereof and at least one pharmaceutically acceptable excipient are preferably contained within the capsule in a solid form. Preferably, this solid form is selected from a powder, granulate, pellets, tablets, thixotropic gel or a mixture thereof. More preferably, this solid form is selected from a powder, granulate or pellets. Even more preferably, this solid form is a powder. Accordingly, it is not necessary to compress and/or tablet the material to be filled into the capsule. On the one hand, this results in a decrease of mechanical stress to which the material is exposed during preparation. On the other hand, omarigliptin or the pharmaceutically acceptable salt thereof and the excipient will not be in as close contact as in the case of a tablet. Without being bound by theory, it can be postulated that the absence of a compression step during capsule manufacture will result in a decrease in the rate of the Maillard reaction, which is known to affect stability and purity of omarigliptin in compositions of the prior art. However, also when granulates or pellets are used as filling material, it is possible to at least reduce compression forces when compared to tableting. Thus, the present invention provides an omarigliptin composition with increased stability and reduced risk of degradation.

In a preferred embodiment of the hard-shelled capsule of the present invention, the at least one pharmaceutically acceptable excipient is selected from the group consisting of diluents such as calcium carbonate, sodium carbonate, kaolin, lactose, mannitol, calcium phosphate or sodium phosphate; granulating agents; binders such as starch, gelatin or acacia; glidants such as magnesium stearate and/or silicon dioxide; wetting agents; fillers; surfactants; anti-oxidants; sweeteners, or any combination thereof.

In an even more preferred embodiment of the hard-shelled capsule of the present invention, the capsule is a hard gelatin capsule and the at least one pharmaceutically acceptable excipient comprises at least one excipient selected from
a) the group consisting of sucrose, mannitol, microcrystalline cellulose, xylitol, maltitol, lactitol, starch, hydroxypropylcellulose (HPC), hydroxypropylmethyl cellulose (HMPC), sorbitol and mixtures thereof,
b) the group consisting of maltodextrin, lactose monohydrate, polyvinylpyrrolidone, silicified microcrystalline cellulose, hydroxyethyl cellulose, anhydrous lactose, mannose, glucose, maltose, other reducing sugars, neusilin, kollidon, crosspovidone, and mixtures thereof, or
c) any combination of the excipients recited under a) and b).

The pharmaceutical composition of the present invention may comprise a glidant. The glidant is, for example, silicon dioxide. Preferably, the glidant is present in the pharmaceutical composition of the present invention in an amount of 0.2 to 2% by weight, more preferably in an amount of 0.5 to 1% by weight, based on the filling weight.

In a specific embodiment of the hard-shelled capsule of the present invention the at least one pharmaceutically acceptable excipient comprises at least one excipient selected from the group consisting of mannitol, silicon dioxide, preferably in the form of fumed silica, lactose, lactose monohydrate, maltodextrin, microcrystalline cellulose, or combinations thereof. In a more specific embodiment, the at least one pharmaceutically acceptable excipient comprises mannitol or maltodextrin, preferably maltodextrin DE 1, and silicon dioxide.

With regard to the capsule size, there is no limitation, provided the capsules are useful for oral administration. Preferably, capsules of standard sizes 1, 2, 3 or 4, are used, e.g. having a volume of about 0.5 ml, 0.37 ml, 0.30 ml and 0.21 ml, respectively, e.g. according to DAC/NRF (German Drug Codex/New German Formulary "Deutscher Arzneimittel-Codex/Neue Rezeptur-Formularium").

When the capsule according to the present invention is a soft-shelled capsule, preferably a soft gelatin capsule, omarigliptin or a pharmaceutically acceptable salt thereof and at least one pharmaceutically acceptable excipient are preferably contained within the capsule in a liquid or semisolid form. Preferably, this liquid form is selected from a solution, suspension or emulsion of omarigliptin or a pharmaceutically acceptable salt thereof. In this context, preferred liquid phases are based on hydrophobic liquids selected from the group consisting of fatty oils, liquid hydrocarbons, medium chained triglycerides (e.g. Miglyol ® 812) and essential oils. Specific non-limiting examples are peanut oil, liquid paraffin or olive oil.

Omarigliptin can be present in the pharmaceutical composition of the present invention in the form of a free base or in the form of a pharmaceutically acceptable salt thereof.

Omarigliptin is preferably present in the pharmaceutical composition according to the present invention in an amount of more than 15% by weight, preferably more than 20% by weight, more preferably more than 25% by weight, even more preferably more than 30% by weight, based on the filling weight. In particular, omarigliptin is preferably present in the pharmaceutical composition according to the present invention in an amount of more than 15% to less than 50% by weight, preferably more than 20% to less than 50% by weight, more preferably more than 25% to less than 50% by weight, even more preferably more than 30% to less than 50% by weight, based on the filling weight.

Omarigliptin can be prepared as described in WO 2010/056708. Omarigliptin exists in several polymorphic forms and in amorphous form. The pharmaceutical composition according to the present invention may comprise omarigliptin in all polymorphic forms or in amorphous form. Preferably, omarigliptin form I or omarigliptin form V is present in the pharmaceutical composition according to the present invention. The polymorphic forms are described, for example, in WO 2013/003249 and in patent application number EP15182400.0

In one embodiment of the present invention, the omarigliptin is in the form of a pharmaceutically acceptable acid addition salt. Pharmaceutically acceptable acid addition salts of omarigliptin include, without limitation, the hydrochloride, nitrate, phosphate, sulfate, acetate, benzoate, citrate, malate, maleate, mesylate, besylate, succinate, tartrate, oxalate, lactate, fumarate and mandelate salt. Preferred pharmaceutically acceptable acid addition salts of omarigliptin are the hydrochloride, nitrate, phosphate, fumarate and mandelate salt. A particularly preferred pharmaceutically acceptable acid addition salt of omarigliptin is the fumarate salt. Another particularly preferred pharmaceutically acceptable acid addition salt of omarigliptin is the mandelate salt (including the omarigliptin R-mandelate, omarigliptin S-mandelate and omarigliptin RS-mandelate). Another particularly preferred pharmaceutically acceptable acid addition salt of omarigliptin is the hydrochloride salt.

In embodiments comprising a pharmaceutically acceptable omarigliptin acid addition salt, the pharmaceutically acceptable omarigliptin acid addition salt is preferably non-hygroscopic, i.e. show a water uptake of at most 2.0 weight%, preferably of at most 1.4 weight%, preferably of at most 1.0 weight%, preferably of at most 0.8 weight%, preferably of at most 0.7%, based on the weight of the omariglipitin acid addition salt, when measured with gravimetric moisture sorption at a relative humidity in the range of from 0 to 95% and a temperature of 25.0 ± 0.1 °C.

The pharmaceutically acceptable acid addition salts of omarigliptin useful in the present invention can be in amorphous form, crystalline form or in a mixture of crystalline and amorphous forms. Preferably, the pharmaceutically acceptable acid addition salts of omarigliptin useful in the present invention are in crystalline form. One preferred crystalline acid addition salt of omarigliptin is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C. Another preferred crystalline acid addition salt of omarigliptin is crystalline omarigliptin fumarate characterized by having a powder X-ray diffractogram comprising reflections at 2-Theta angles selected from the group of (13.8 ± 0.2)°, (19.7 ± 0.2)° and (21.2 ± 0.2)°, when measured with Cu-Kalpha_{1,2} radiation at a wavelength of 0.15419 nm and a temperature in the range of from 20 to 30 °C. Another preferred crystalline acid addition salt of omarigliptin is crystalline omarigliptin (R)-mandelate characterized by having a powder X-ray diffractogram comprising reflections at 2-Theta angles selected from the group of (6.3 ± 0.2)°, (17.9 ± 0.2)° and (21.9 ± 0.2)°, when measured with Cu-Kalpha_{1,2} radiation at a wavelength of 0.15419 nm and a temperature in the range of from 20 to 30 °C. Another preferred crystalline acid addition salt of omarigliptin is crystalline omarigliptin (S)-mandelate characterized by having a powder X-ray diffractogram comprising reflections at 2-Theta angles selected from the group of (16.3 ± 0.2)°, (18.6 ± 0.2)° and (20.7 ± 0.2)°, when measured with Cu-Kalpha_{1,2} radiation at a wavelength of 0.15419 nm and a temperature in the range of from 20 to 30 °C. Another preferred crystalline acid addition salt of omarigliptin is crystalline omarigliptin (R, S)-mandelate characterized by having a powder X-ray diffractogram comprising reflections at 2-Theta angles selected from the group of (6.3 ± 0.2)°, (12.7 ± 0.2)° and (18.6 ± 0.2)°, when measured with Cu-Kalpha_{1,2} radiation at a wavelength of 0.15419 nm and a temperature in the range of from 20 to 30 °C. A particularly preferred crystalline acid addition salt of omarigliptin is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.

In various embodiments, the at least one pharmaceutically acceptable excipient comprises neutral excipients. Neutral excipients are pharmaceutically acceptable excipients which do not have an ionic charge. Non-limiting examples are diluents, such as microcrystalline cellulose and mannitol. Preferably, the neutral excipients comprise microcrystalline cellulose, mannitol or a mixture of microcrystalline cellulose and mannitol.

Thus, in one embodiment of the present invention, the at least one pharmaceutically acceptable excipient comprises neutral excipients, wherein the neutral excipients are present in an amount of at least 75% by weight, based on the filling weight, and wherein the neutral excipients comprise at least one non-reducible sugar diluent. More preferably, the at least one pharmaceutically acceptable excipient comprises neutral excipients wherein the neutral excipients are present in an amount of at least 80% by weight, based on the filling weight, wherein the neutral excipients comprise at least one non-reducible sugar diluent. Even more preferably, the neutral excipients are present in an amount of at least 84% by weight, based on the filling weight.

In another embodiment of the present invention, the at least one pharmaceutically acceptable excipient comprises neutral excipients, wherein the neutral excipients are present in an amount of less than 75% by weight, based on the filling weight. Preferably, the neutral excipients are present in an amount of less than 70% by weight, preferably in an amount of less than 65% by weight, based on the filling weight.

The pharmaceutical composition according to the present invention, especially the pharmaceutical composition of any of the embodiments described above, may additionally comprise one or more non-neutral excipients. Non-neutral excipients are pharmaceutically acceptable excipients which have an ionic charge, such as salts. The non-neutral excipients include, for example, disintegrants and lubricants.

Preferably, the combined amount of neutral excipients and non-neutral excipients is less than 85% by weight, preferably less than 80% by weight, more preferably less than 75% by weight, even more preferably less than 70% by weight, based on the filling weight.

In another embodiment of the present invention, the at least one pharmaceutically acceptable excipient comprises a diluent, wherein the diluent comprises a reducing sugar, and wherein the reducing sugar is maltodextrin.

Maltodextrin is a saccharide mixture of polymers that consist of D-glucose units connected in chains of variable length. Maltodextrin is prepared by partial hydrolysis of starch.

Maltodextrin is a reducible sugar, it gives a positive reaction in the Fehling test, i.e. maltodextrin reduces Fehling's solution.

Maltodextrin usually has a dextrose equivalent (DE) of 1 to 20. Preferably, maltodextrin having a dextrose equivalent of 3 to 20, more preferably maltodextrin having a dextrose equivalent of 5 to 10, and even more preferably maltodextrin having a dextrose equivalent of 3 to 5 is used. The dextrose equivalent is a measure of the extent of polymer hydrolysis and is expressed in grams of D-glucose per 100 g of the dry substance. Preferably, the maltodextrin is the only reducing sugar present in the pharmaceutical composition. Also preferably, the pharmaceutical composition is free of mannitol or free of microcrystalline cellulose. More preferably, the pharmaceutical composition is free of mannitol and free of microcrystalline cellulose.

Regarding the amount of maltodextrin present in the composition, no limitation exists provided suitable compositions are obtained. Preferably, the maltodextrin is present in the pharmaceutical composition in an amount of 60 to 99% by weight, preferably 60 to 90% by weight, more preferably 60 to 80% by weight, even more preferably 60 to 75% by weight, based on the filling weight.

In any of the embodiments described above, the omarigliptin or pharmaceutically acceptable salt thereof is preferably present in an amount of 12.5 mg or 25 mg, calculated as a free base.

Preferably, in the pharmaceutical composition of the present invention less than 0.5% by weight of total impurities are formed under accelerated stability test conditions of 50°C/75% relative humidity in a sealed vial or in an open vial after one week.

The capsules according to the present invention can be prepared according to any suitable process known in the pharmaceutical field. For example, capsules can be prepared by mixing omarigliptin or a pharmaceutically acceptable salt of omarigliptin with one or more excipients, optionally sieving the mixture, and filling the mixture into capsules, for example into hard gelatin capsules.

The pharmaceutical composition of the present invention can be used in the treatment of metabolic diseases, in particular in the treatment of diabetes mellitus type 2.

The present invention is not only useful in a method of treatment with omarigliptin as a single active substance, but also in combination with other therapeutic agents. With regard to such combination therapies, providing two or more therapeutic agents in a single pharmaceutical composition is highly preferred since it requires a patient to take only one composition instead of two or more, and thereby increases patient compliance. Thus, the present invention also encompasses pharmaceutical compositions comprising omarigliptin or a pharmaceutically acceptable salt thereof, at least one pharmaceutically acceptable excipient, and at least one further therapeutic agent, wherein the pharmaceutical composition is in the form of a capsule for oral administration.

Without being intended to limit the scope of combination products encompassed by the present invention, the other therapeutic agents can be selected from e.g. insulin sensitizers, insulin and insulin analogs or derivatives, leptin and leptin derivatives, agonists, and analogs, sulfonylurea and non-sulfonylurea insulin secretagogues, glucagon receptor antagonists, incretin mimetics, LDL cholesterol lowering, HDL-raising drugs, agents intended for use in inflammatory conditions, antihypertensive agents, glucokinase activators, inhibitors of 11 β-hydroxysteroid dehydrogenase type 1, inhibitors of cholesteryl ester transfer protein, inhibitors of fructose 1,6-bisphosphatase, AMP-activated Protein Kinase (AMPK) activators, agonists of the G-protein-coupled receptors, neuromedin U receptor 1 (NMURI) and/or neuromedin U receptor 2 (NMUR2) agonists, GPR- 105 (P2YR14) antagonists, inhibitors of glucose uptake, inhibitors of fatty acid synthase, inhibitors of acyl coenzyme A:monoacylglycerol acyltransferase 1 and 2 (MGAT-1 and MGAT-2), agonists of the TGR5 receptor, bromocriptine mesylate and rapid-release formulations thereof, histamine H3 receptor agonists, and/or α2-adrenergic or β3-adrenergic receptor agonists, e.g., as disclosed in WO 2010/056708 A1.

The increased flexibility with regard to the choice of excipient and the amount of drug loading provided by the present invention is especially advantageous for the provision of such combination products.

The present invention is further illustrated by the following embodiments and combinations of embodiments as indicated by the respective dependencies and references.
1. A pharmaceutical composition comprising
   omarigliptin or a pharmaceutically acceptable salt thereof and
   at least one pharmaceutically acceptable excipient,
   wherein the pharmaceutical composition is in the form of a capsule for oral administration.
2. Pharmaceutical composition according to embodiment 1, wherein the capsule is a hard-shelled or soft-shelled capsule.
3. Pharmaceutical composition according to any one of the preceding embodiments, wherein the capsule is a hard-shelled capsule, preferably a hard gelatin capsule, containing each the omarigliptin or pharmaceutically acceptable salt thereof and the at least one pharmaceutically acceptable excipient in a solid form.
4. Pharmaceutical composition according to embodiment 3, wherein the solid form is a powder, granulate, pellets, tablets, thixotropic gel or a mixture thereof.
5. Pharmaceutical composition according to any one of the preceding embodiments, wherein the at least one pharmaceutically acceptable excipient is selected from the group consisting of diluents, granulating agents, binders, glidants, wetting agents, fillers, surfactants, anti-oxidants, sweeteners, or a combination thereof.
6. Pharmaceutical composition according to any one of the preceding embodiments, wherein the at least one pharmaceutically acceptable excipient comprises at least one excipient selected from
   a) the group consisting of sucrose, mannitol, microcrystalline cellulose, xylitol, maltitol, lactitol, starch, hydroxypropylcellulose (HPC), hydroxypropylmethyl cellulose (HMPC), sorbitol and mixtures thereof,
   b) the group consisting of maltodextrin, lactose, lactose monohydrate, polyvinylpyrrolidone, silicified microcrystalline cellulose, hydroxyethyl cellulose, anhydrous lactose, mannose, glucose, maltose, other reducing sugars, Neusilin, kollidon, crosspovidone, and mixtures thereof, or
   c) any combination of the excipients recited under a) and b).
7. Pharmaceutical composition according to any one of the preceding embodiments, wherein the at least one pharmaceutically acceptable excipient comprises an excipient selected from the group consisting of mannitol, silicon dioxide, preferably in the form of fumed silica, lactose, maltodextrin, microcrystalline cellulose, or combinations thereof.
8. Pharmaceutical composition according to any one of the preceding embodiments, wherein the at least one pharmaceutically acceptable excipient comprises maltodextrin and silicon dioxide.
9. Pharmaceutical composition according to any one of embodiments 1-2, wherein the capsule is a soft-shelled capsule, preferably a soft gelatin capsule, containing the omarigliptin or pharmaceutically acceptable salt thereof and the at least one pharmaceutically acceptable excipient in a liquid or semisolid form.
10. Pharmaceutical composition according to embodiment 9, wherein the liquid form is a solution, suspension or emulsion of omarigliptin or a pharmaceutically acceptable salt thereof, preferably in a hydrophobic liquid selected from the group consisting of fatty oils, liquid hydrocarbons, medium chained triglycerides (e.g. Miglyol ® 812) and essential oils.
11. The pharmaceutical composition according to any of embodiments 1 to 10, wherein the pharmaceutically acceptable salt of omarigliptin is a pharmaceutically acceptable acid addition salt.
12. The pharmaceutical composition according embodiment 11, wherein the pharmaceutically acceptable acid addition salt is selected from the group consisting of omarigliptin hydrochloride, omarigliptin sulfate, omarigliptin acetate, omarigliptin benzoate, omarigliptin citrate, omarigliptin malate, omarigliptin maleate, omarigliptin mesylate, omarigliptin besylate, omarigliptin succinate, omarigliptin tartrate, omarigliptin oxalate, omarigliptin lactate, omarigliptin mandelate, omarigliptin fumarate, omarigliptin nitrate and omarigliptin phosphate.
13. The pharmaceutical composition according to any one of embodiments 11 or 12, wherein the pharmaceutically acceptable acid addition salt is omarigliptin hydrochloride.
14. The pharmaceutical composition according to any of embodiments 11 to 13, wherein the omarigliptin acid addition salt shows a water uptake of at most 2.0 weight%, preferably of at most 1.4 weight%, preferably of at most 1.0 weight%, preferably of at most 0.8 weight%, preferably of at most 0.7%, based on the weight of the omariglipitin acid addition salt, when measured with gravimetric moisture sorption at a relative humidity in the range of from 0 to 95% and a temperature of 25.0 ± 0.1 °C.
15. The pharmaceutical composition according to any of embodiments 11 to 14, wherein the omarigliptin acid addition salt is in amorphous form, crystalline form or in a mixture of crystalline and amorphous forms.
16. The pharmaceutical composition according to embodiment 13, wherein the omarigliptin acid addition salt is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.
17. The pharmaceutical composition according to any of embodiments 1 to 16, wherein the at least one pharmaceutically acceptable excipient comprises neutral excipients.
18. The pharmaceutical composition according to any of embodiments 1 to 17, wherein the at least one pharmaceutically acceptable excipient comprises neutral excipients and wherein the neutral excipients are present in an amount of at least 75% by weight, based on the capsule filling weight, wherein the neutral excipients comprise at least one non-reducible sugar diluent.
19. The pharmaceutical composition according to embodiment 18, wherein the neutral excipients are present in an amount of at least 80%, preferably at least 84% by weight, based on the capsule filling weight.
20. The pharmaceutical composition according to any of embodiments 1 to 17, wherein the at least one pharmaceutically acceptable excipient comprises neutral excipients, wherein the neutral excipients are present in an amount of less than 75% by weight, based on the capsule filling weight.
21. The pharmaceutical composition according to embodiment 20, wherein the neutral excipients are present in an amount of less than 70% by weight, preferably in an amount of less than 65% by weight, based on the capsule filling weight.
22. The pharmaceutical composition according to any of embodiments 17 to 21, wherein the neutral excipients comprise microcrystalline cellulose.
23. The pharmaceutical composition according to any of embodiments 17 to 21, wherein the neutral excipients comprise mannitol.
24. The pharmaceutical composition according to any of embodiments 17 to 21, wherein the neutral excipients comprise microcrystalline cellulose and mannitol.
25. The pharmaceutical composition according to any of embodiments 17 to 24 further comprising at least one non-neutral excipient.
26. The pharmaceutical composition according to any of embodiments 20 to 25, wherein the combined amount of neutral excipients and non-neutral excipients is less than 85% by weight, preferably less than 80% by weight, more preferably less than 75% by weight, even more preferably less than 70% by weight, based on the capsule filling weight.
27. The pharmaceutical composition according to any of embodiments 1 to 17, wherein the at least one pharmaceutically acceptable excipient comprises a diluent, wherein the diluent comprises a reducing sugar, and wherein the reducing sugar is maltodextrin.
28. The pharmaceutical composition according to embodiment 27, wherein the maltodextrin has a dextrose equivalent of 1 to 20, preferably 3 to 20, more preferably 5 to 10.
29. The pharmaceutical composition according to any of embodiments 27 or 28, wherein the maltodextrin is the only reducing sugar present in the pharmaceutical composition.
30. The pharmaceutical composition according to any of embodiments 27 to 29, wherein the maltodextrin is the only diluent present in the pharmaceutical composition.
31. The pharmaceutical composition according to any of embodiments 27 to 30, wherein the pharmaceutical composition is free of mannitol.
32. The pharmaceutical composition according to any of embodiments 27 to 31, wherein the pharmaceutical composition is free of microcrystalline cellulose.
33. The pharmaceutical composition according to any of embodiments 27 to 32, wherein the pharmaceutical composition is free of mannitol and free of microcrystalline cellulose.
34. The pharmaceutical composition according to any of embodiments 27 to 33, wherein the maltodextrin is present in the pharmaceutical composition in an amount of 60 to 99% by weight, preferably 60 to 90% by weight, more preferably 60 to 80% by weight, even more preferably 60 to 75% by weight, based on the capsule filling weight.
35. The pharmaceutical composition according to any of embodiments 1 to 34, wherein the omarigliptin or pharmaceutically acceptable salt thereof is present in an amount of 12.5 mg or 25 mg, calculated as a free base.
36. The pharmaceutical composition according to any of embodiments 1 to 35, comprising at least one further therapeutic agent.
37. The pharmaceutical composition according to any of embodiments 1 to 36 for use in the treatment of diabetes mellitus type 2.

### EXAMPLES

The following examples illustrate the present invention and are not intended to limit the scope of the invention set forth in the claims appended hereto.

### Example 1: Hard gelatin capsule

| Component | Amount (mg) | Amount (% by weight) | Amount (mg) | Amount (% by weight) |
|---|---|---|---|---|
| Omarigliptin | 25 | 29.24 | 12.5 | 29.24 |
| Maltodextrin | 60 | 70.18 | 30 | 70.18 |
| Silicon dioxide | 0.5 | 0.58 | 0.25 | 0.58 |
| Filling weight | 85.50 | 100.00 | 42.75 | 100.00 |
| Sum excipients | 60.5 | 70.76 | 30.25 | 70.76 |

All components in the amounts mentioned in the above table are mixed, passed through a 1.0 mm sieve and filled into hard gelatin capsules.

### Example 2: Hard gelatin capsule

| Component | Amount (mg) | Amount (% by weight) | Amount (mg) | Amount (% by weight) |
|---|---|---|---|---|
| Omarigliptin HCl | 27.2 | 31.04 | 13.6 | 31.04 |
| Maltodextrin | 60 | 68.42 | 30 | 68.42 |
| Silicon dioxide | 0.5 | 0.57 | 0.25 | 0.57 |
| Filling weight | 87.70 | 100.00 | 43.85 | 100.00 |
| Sum excipients | 60.5 | 68.99 | 30.25 | 68.99 |

All components in the amounts mentioned in the above table are mixed, passed through a 1.0 mm sieve and filled into hard gelatin capsules.

### Example 3: Hard gelatin capsule

| Component | Amount (mg) | Amount (% by weight) | Amount (mg) | Amount (% by weight) |
|---|---|---|---|---|
| Omarigliptin | 25 | 29.24 | 12.5 | 29.24 |
| Mannitol | 60 | 70.18 | 30 | 70.18 |
| Silicon dioxide | 0.5 | 0.58 | 0.25 | 0.58 |
| Filling weight | 85.50 | 100.00 | 42.75 | 100.00 |
| Sum excipients | 60.5 | 70.76 | 30.25 | 70.76 |

All components in the amounts mentioned in the above table are mixed, passed through a 1.0 mm sieve and filled into hard gelatin capsules.

### Example 4: Hard gelatin capsule

| Component | Amount (mg) | Amount (% by weight) | Amount (mg) | Amount (% by weight) |
|---|---|---|---|---|
| Omarigliptin | 25 | 29.24 | 12.5 | 29.24 |
| Lactose Monohydrate | 60 | 70.18 | 30 | 70.18 |
| Silicon dioxide | 0.5 | 0.58 | 0.25 | 0.58 |
| Filling weight | 85.50 | 100.00 | 42.75 | 100.00 |
| Sum excipients | 60.5 | 70.76 | 30.25 | 70.76 |

All components in the amounts mentioned in the above table are mixed, passed through a 1.0 mm sieve and filled into hard gelatin capsules.

### Example 5: Hard gelatin capsule

| Component | Amount (mg) | Amount (% by weight) | Amount (mg) | Amount (% by weight) |
|---|---|---|---|---|
| Omarigliptin | 25 | 29.24 | 12.5 | 29.24 |
| Microcrystalline Cellulose | 60 | 70.18 | 30 | 70.18 |
| Silicon dioxide | 0.5 | 0.58 | 0.25 | 0.58 |
| Filling weight | 85.50 | 100.00 | 42.75 | 100.00 |
| Sum excipients | 60.5 | 70.76 | 30.25 | 70.76 |

All components in the amounts mentioned in the above table are mixed, passed through a 1.0 mm sieve and filled into hard gelatin capsules.

## Claims

1. A pharmaceutical composition comprising
omarigliptin or a pharmaceutically acceptable salt thereof and
at least one pharmaceutically acceptable excipient,
wherein the pharmaceutical composition is in the form of a capsule for oral administration.

2. Pharmaceutical composition according to claim 1, wherein the capsule is a hard-shelled or soft-shelled capsule.

3. Pharmaceutical composition according to any one of the preceding claims, wherein the capsule is a hard-shelled capsule, preferably a hard gelatin capsule, containing each the omarigliptin or pharmaceutically acceptable salt thereof and the at least one pharmaceutically acceptable excipient in a solid form, preferably wherein the solid form is a powder, granulate, pellets, tablets, thixotropic gel or a mixture thereof.

4. Pharmaceutical composition according to any one of the preceding claims, wherein the at least one pharmaceutically acceptable excipient comprises an excipient selected from the group consisting of mannitol, silicon dioxide, preferably in the form of fumed silica, lactose, maltodextrin, microcrystalline cellulose, or combinations thereof.

5. Pharmaceutical composition according to any one of claims 1 to 2, wherein the capsule is a soft-shelled capsule, preferably a soft gelatin capsule, containing the omarigliptin or pharmaceutically acceptable salt thereof and the at least one pharmaceutically acceptable excipient in a liquid or semisolid form, preferably wherein the liquid form is a solution, suspension or emulsion of omarigliptin or a pharmaceutically acceptable salt thereof, preferably in a hydrophobic liquid selected from the group consisting of fatty oils, liquid hydrocarbons, medium chained triglycerides (e.g. Miglyol ® 812) and essential oils.

6. The pharmaceutical composition according to any of claims 1 to 5, wherein the pharmaceutically acceptable salt of omarigliptin is a pharmaceutically acceptable acid addition salt, preferably wherein the pharmaceutically acceptable acid addition salt is omarigliptin hydrochloride.

7. The pharmaceutical composition according to any of claims 1 to 6, wherein the at least one pharmaceutically acceptable excipient comprises neutral excipients and wherein the neutral excipients are present in an amount of at least 75%, preferably at least 80%, more preferably at least 84% by weight, based on the capsule filling weight, wherein the neutral excipients comprise at least one non-reducible sugar diluent.

8. The pharmaceutical composition according to any of claims 1 to 6, wherein the at least one pharmaceutically acceptable excipient comprises neutral excipients, wherein the neutral excipients are present in an amount of less than 75%, preferably less than 70%, more preferably less than 65% by weight, based on the capsule filling weight.

9. The pharmaceutical composition according to any of claims 1 to 6, wherein the at least one pharmaceutically acceptable excipient comprises a diluent, wherein the diluent comprises a reducing sugar, and wherein the reducing sugar is maltodextrin.

10. The pharmaceutical composition according to claim 9, wherein the maltodextrin is present in the pharmaceutical composition in an amount of 60 to 99% by weight, preferably 60 to 90% by weight, more preferably 60 to 80% by weight, even more preferably 60 to 75% by weight, based on the capsule filling weight.

11. The pharmaceutical composition according to any of claims 1 to 10, wherein the omarigliptin or pharmaceutically acceptable salt thereof is present in an amount of 12.5 mg or 25 mg, calculated as a free base.

12. The pharmaceutical composition according to any of claims 1 to 11, comprising at least one further therapeutic agent.

13. The pharmaceutical composition according to any of claims 1 to 12 for use in the treatment of diabetes mellitus type 2.
